# FASCICULE DE BREVET EUROPEEN

(11) **EP 0 773 835 B1**
(45) Date de publication et mention de la délivrance du brevet: **27.05.1998**
(21) Numéro de dépôt: 94904665.0
(22) Date de dépôt: 12.01.1994
(51) Int. Cl.: B05B 11/06, A61M 15/00

(54) **DISPOSITIF PORTATIF POUR PROJETER DES DOSES D'UNE SUBSTANCE FLUIDE A L'AIDE D'UN FLUX D'AIR COMPRIME**
TRAGBARE VORRICHTUNG ZUM SPRITZEN EINER FLIESSFÄHIGEN SUBSTANZ MITTELS DRUCKLUFT
SPRAY DEVICE FOR DISPENSING DOSES OF A FLUID SUBSTANCE BY MEANS OF A COMPRESSED AIR FLOW

(30) Priorité: 14.01.1993 FR 9300309
(43) Date de publication de la demande: 21.05.1997
(73) Titulaire: VALOIS SA, 27110 Le Neubourg (FR)
(72) Inventeur: BRUNA, Pascal, F-76000 Rouen (FR)
(74) Mandataire: Pinguet, André
(86) Numéro de dépôt international: FR9400035
(87) Numéro de publication internationale: WO9415716

(56) Documents cités:
- EP-A- 0 473 965
- WO-A-92/04068
- US-A- 5 113 855

## Description

La présente invention concerne un dispositif portatif pour projeter des doses d'une substance fluide à l'aide d'un flux d'air comprimé.

Les substances fluides visées sont en particulier des poudres mais aussi éventuellement des liquides.

La présente invention s'applique particulièrement au domaine des pulvérisateurs destinés à projeter des doses de substance pharmaceutique en poudre sur les muqueuses des voies respiratoires (voir orale ou voie nasale). Ces pulvérisateurs utilisent un effet de projection de la poudre sur les muqueuses, au contraire des inhalateurs destinés à envoyer la poudre dans les bronches, dans lesquels la poudre est transportée par le flux d'air inhalé par le patient lorsqu'il respire.

Il est connu d'utiliser dans ce but des pulvérisateurs aérosols. La poudre est contenue dans un réservoir avec un gaz propulseur liquéfié, et une valve déclenche l'expulsion de la poudre en suspension dans le gaz liquéfié. En se détendant à la pression atmosphérique, le gaz liquéfié repasse sous forme gazeuse en se dilatant fortement, ce qui propulse la poudre.

Mais les aérosols présentent l'inconvénient d'utiliser des gaz propulseurs dangereux soit pour l'environnement (fréons), soit pour les utilisateurs (hydrocarbures). En outre, ils nécessitent d'agiter correctement le réservoir avant d'expulser la poudre, afin de la mettre en suspension dans le gaz liquéfié. Si un utilisateur oublie d'agiter le réservoir, ou l'agite insuffisamment, la dose de poudre expulsée est imprécise.

Le document US-5,113,855 décrit un inhalateur de poudre dans lequel un organe d'actionnement est déplaçable entre une position de repos et d'actionnement pour amener d'une part une dose de poudre dans un godet, aménagé dans le canal d'éjection, à partit d'une réserve et comprimer d'autre part de l'air qui pourra s'échapper au niveau du godet contenant la poudre. Cependant, le flux d'air comprimé ne sert qu'à vider le godet et non à propulser la poudre hors de l'inhalateur dans la bouche ou les poumons de l'utilisateur. Il s'ensuit que de la poudre peut rester dans l'inhalateur.

La présente invention a pour but de remédier à ces inconvénients.

La présente invention a pour objet un dispositif pour projeter des doses d'une substance fluide à l'aide d'un flux d'air comprimé, ledit dispositif comprenant :
- un corps ayant un canal d'éjection,
- un organe d'actionnement déplaçable par rapport au corps entre une position de repos et une position d'actionnment,
- un organe élastique de rappel de l'organe d'actionnement, qui sollicite l'organe d'actionnement vers sa position de repos,
- une réserve destinée à contenir ladite substance,
- des moyens de chargement adaptés à déplacer une dose de ladite substance hors de la réserve jusque dans le canal d'éjection lorsque l'organe d'actionnement est déplacé de sa position de repos jusqu'à sa position d'actionnement,
- une pompe à air ayant une chambre de pompe dotée d'une paroi déplaçable solidaire de l'organe d'actionnement et adaptée à comprimer la chambre de pompe lorsque l'organe d'actionnement est déplacé depuis sa position de repos vers sa position d'actionnement, ladite chambre de pompe ayant un clapet de sortie qui communique avec le canal d'éjection, le clapet de sortie étant constitué par un organe d'obturation collaborant avec une ouverture de sortie, ledit organe d'obturation isolant l'ouverture de sortie par rapport à la chambre de pompe tant que l'organe d'actionnement n'est pas au voisinage immédiat de sa position d'actionnement, ledit organe d'obturation ouvrant la communication entre la chambre de pompe et l'ouverture de sortie lorsque l'organe d'actionnement est au voisinage immédiat de sa position d'actionnement,
   caractérisé en ce que ladite chambre de pompe a un clapet d'entrée qui communique avec l'atmosphère et en ce que l'organe d'obturation le clapet de sortie est constitué par un piston de soupape solidaire en déplacement de l'organe d'actionnement et coulissant avec étanchéité dans un cylindre de soupape ayant une extrémité supérieure, ledit cylindre ayant une ouverture de sortie qui communique avec le canal d'éjection, ledit cylindre de soupape ayant son extrémité qui communique avec la chambre de pompe, ladite ouverture de sortie étant positionnée suffisamment éloignée ladite extrémité du cylindre de soupape pour que le piston de soupape isole l'ouverture de sortie par rapport à la chambre de pompe tant que l'organe d'actionnement n'est pas au voisinage immédiat de sa position d'actionnement.

Selon une forme de réalisation de l'invention, le cylindre de soupape peut posséder un orifice d'évent positionné de façon que ladite ouverture de sortie soit disposée entre ledit orifice d'évent et ladite extrémité du cylindre de soupape qui communique avec la chambre de pompe. Dans ce cas, ledit orifice d'évent peut éventuellement être muni d'un filtre pour empêcher la pénétration de poussières dans le cylindre de soupape.

Selon une autre caractéristique de l'invention, les moyens de chargement sont constitués par une tige de chargement, ayant un évidement de dosage à une position intermédiaire sur sa longueur, la tige de chargement n'est pas solidaire de l'organe d'actionnement, ladite tige de chargement est sollicitée vers une position de butée par un moyen élastique et l'organe d'actionnement parcourt une certaine course depuis sa position de repos avant de repousser ladite tige contre l'action du moyen élastique de la tige de chargement.

Des exemples de dispositifs de chargement de doses avec un organe mobile doté d'un ou plusieurs évidements de dosage sont donnés dans les documents WO-A-92 05823, EP-A-0 069 715 (et brevet américain correspondant US-A-4 524 769), GB-A-2 041 763, EP-A-0 166 294, GB-A-2 165 159, EP-A-0 079 478, US-A-2 587 215 et US-A-2 581 182.

Selon une forme de réalisation particulière, l'évidement de dosage a une forme annulaire et se trouve compris dans le canal d'éjection lorsque l'organe d'actionnement est dans sa position d'actionnement.

Selon une forme de réalisation avantageuse, la tige de chargement coulisse dans la réserve de substance à projeter, ladite réserve est une enceinte coulissant à mouvement perdu parallèlement à la tige de chargement dans un logement du corps, un moyen élastique sollicite ladite enceinte de façon à l'éloigner du canal d'éjection, l'enceinte possède une paroi éloignée du canal d'éjection, qui comporte un orifice traversé par la tige de chargement, la tige de chargement comporte un relief latéral qui se trouve à l'extérieur de l'enceinte lorsque l'organe d'actionnement est dans sa position de repos, ledit relief latéral interfère avec ladite paroi de l'enceinte en déplaçant l'enceinte sur une certaine distance contre la sollicitation du moyen élastique de l'enceinte lorsque l'organe d'actionnement est déplacé vers sa position d'actionnement, et ledit relief a une souplesse suffisante pour s'effacer élastiquement en traversant ledit orifice de ladite paroi de l'enceinte, lorsqu'une force axiale suffisante est exercée par ledit relief latéral sur ladite paroi de l'enceinte. Un dispositif de chargement de dose de poudre ayant une tige de chargement et une enceinte coulissante est déjà connu du document WO-A-92 05823.

En variante, ladite paroi de l'enceinte qui est qui est éloignée du canal d'éjection, a une souplesse suffisante pour se déformer élastiquement de façon à laisser le relief latéral de la tige de chargement traverser l'orifice de ladite paroi, lorsqu'une force axiale suffisante est exercée par ledit relief latéral sur ladite paroi de l'enceinte.

D'autres caractéristiques et avantages apparaîtront au cours de la description détaillée d'une forme de réalisation de l'invention, donnée à titre d'exemple non limitatif, en regard des dessins joints.

Sur les dessins :
- la figure 1 est une vue en coupe verticale longitudinale d'une première forme de réalisation du dispositif de l'invention,
- la figure 2 est une vue partielle représentant la partie droite de la figure 1,
- la figure 3 est une vue partielle représentant la partie gauche de la figure 1,
- la figure 3a est une vue de détail d'une variante du dispositif de la figure 1,
- la figure 4 est vue d'une variante de la partie gauche de la figure 1,
- la figure 5 est une vue schématique en coupe verticale longitudinale d'une variante du dispositif de la figure 1, et
- la figure 6 est une vue schématique en coupe verticale tranversale d'une variante du dispositif de la figure 5.

Sur les différentes figures, les parties identiques ou similaires sont désignées par les mêmes références.

Le dispositif de l'invention peut généralement être réalisé à l'aide des pièces moulées en matière plastique, à l'exception des ressorts qui sont généralement métalliques et des joints d'étanchéité, généralement en matériau élastomère. Dans la description qui suit, on ne décrira donc généralement pas le matériau constitutif des différentes pièces du dispositif de l'invention, sauf lorsque ce matériau présentera des particularités.

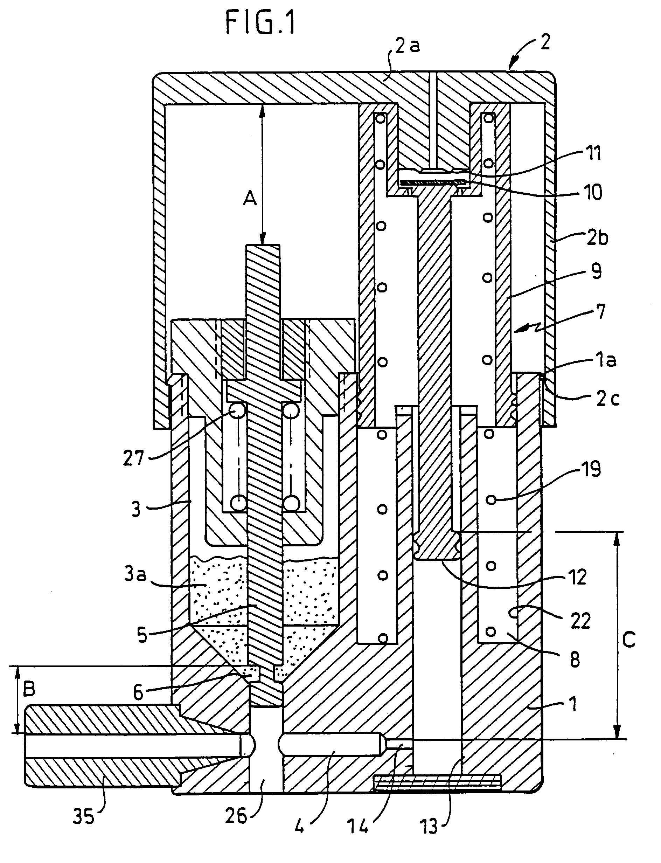

En référence à la figure 1, le dispositif de l'invention se présente extérieurement sous la forme d'un boîtier adapté à être tenu à la main. Ce boîtier se compose d'un corps 1 et d'un capot ou poussoir 2, qui recouvre le corps 1 et qui coulisse axialement par rapport au corps 1 entre une position de repos, représentée sur la figure 1, et une position d'actionnement dans laquelle le poussoir 2 est rapproché au maximum du corps 1. Un ressort de rappel 19 sollicite le poussoir 2 vers sa position de repos.

Le capot ou poussoir 2 comporte une paroi supérieure horizontale 2a, et une paroi latérale verticale 2b qui s'étend vers le bas jusqu'à une extrémité ayant un rebord intérieur 2c. Le rebord intérieur 2c collabore avec des ergots ou des reliefs exterieurs 1a du corps pour empêcher que la paroi latérale 2a ne se dégage du corps 1 sous l'effet du ressort de rappel 19. Le rebord 2c et les reliefs 1a définissent ainsi la position de repos du poussoir 2.

Le corps 1 comporte une réserve 3 de substance destinée à être projetée dans un flux d'air comprimé. Dans l'exemple représenté sur la figure 1, ladite substance est une poudre 3a, ce qui représente le cas le plus courant, bien qu'il soit possible de projeter aussi un liquide avec le dispositif de l'invention.

Le corps 1 comporte en outre un canal d'éjection 4, et un embout 35 qui prolonge le canal d'éjection à l'extérieur du corps 1. L'embout 35 pourrait avoir des formes diverses suivant les utilisations auxquelles est destiné le dispositif, par exemple la pulvérisation nasale ou la pulvérisation orale de la poudre.

Par ailleurs, le dispositif de la figure 1 comporte des moyens de chargement, qui seront décrits en détail ci-après, et qui sont adaptés à déplacer une dose de poudre depuis la réserve 3 jusque dans le canal d'éjection 4 lorsque le poussoir est déplacé depuis sa position de repos jusqu'à sa position d'actionnement.

Enfin, le dispositif comporte une pompe à air 7, dont la sortie communique avec le canal d'éjection 4, et qui est adaptée à envoyer dans ledit canal 4 un flux d'air comprimé lorsque le poussoir 2 arrive dans sa position d'actionnement.

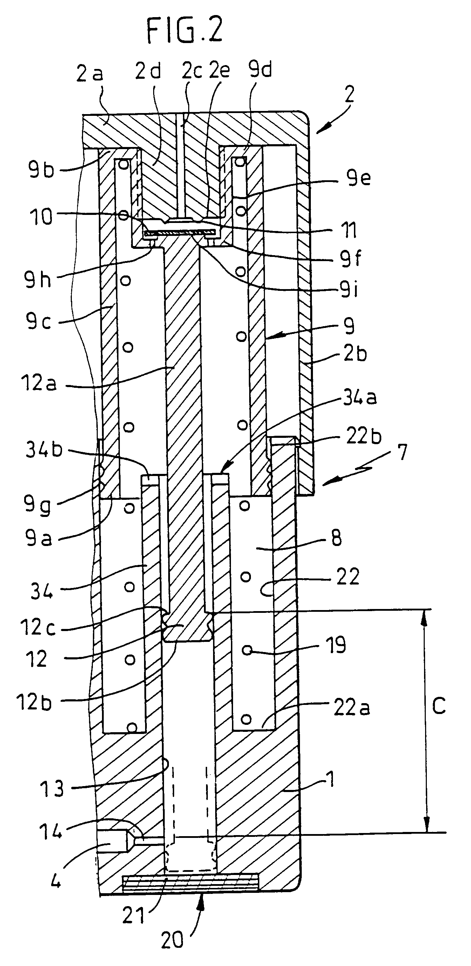

Comme représenté plus en détail sur la figure 2, la pompe à air 7 du dispositif de la figure 1 comporte un cylindre de pompe 22 formé dans le corps 1, ledit cylindre de pompe 22 s'étendant verticalement entre un fond 22a et une extrémité supérieure ouverte 22b, proche du poussoir 2. La pompe 7 comporte en outre un piston de pompe 9, qui coulisse avec étanchéité dans le cylindre de pompe 22, le cylindre 22 et le piston 9 définissant ensemble une chambre de pompe 8 remplie d'air.

Dans l'exemple représenté sur la figure 2, le piston de pompe 9 est tubulaire. Le piston de pompe 9 comporte une paroi cylindrique extérieure 9c, qui s'étend entre une extrémité inférieure 9a et une extrémité supérieure 9b. L'extrémité inférieure 9a comporte avantageusement une ou plusieurs lèvres périphériques d'étanchéité 9g, qui coulissent avec étanchéité dans le cylindre de pompe 22. L'extrémité supérieure 9b de la paroi cylindrique 9c se prolonge radialement vers l'intérieur par un rebord annulaire 9d, qui lui-même se prolonge radialement vers l'intérieur par une cuvette formée par une paroi cylindrique intérieure 9e qui s'étend verticalement vers le bas à partir du rebord 9d jusqu'à un fond central 9f. La paroi supérieure 2a du poussoir 2 comporte une partie cylindrique 2b qui s'étend verticalement vers le bas jusqu'à une face d'extrémité 2e. La partie cylindrique 2b est engagée à l'intérieur de la paroi cylindrique intérieure 9e du piston, les deux pièces étant fixées l'une à l'autre par emboîtement à force ou par un autre moyen. Comme représenté sur la figure 2, la partie cylindrique peut éventuellement être filetée extérieurement tandis que la paroi cylindrique intérieure 9e du piston est filetée intérieurement, les deux pièces étant ainsi vissées l'une dans l'autre. Après assemblage, le rebord annulaire 9d du piston est en butée contre la paroi supérieure 2a du poussoir, et un espace est laissé libre entre le fond 9h et la face d'extrémité 2e. Le poussoir 2 comporte un trou d'entrée d'air 2c, qui est percé verticalement au centre de la partie cylindrique 2d, et qui débouche d'une part dans l'espace compris entre le fond 9f et la face d'extrémité 2e, d'autre part à l'atmosphère.

Par ailleurs, le fond 9f comporte une surélévation 9i ainsi qu'un ou plusieurs orifices 9h qui font communiquer l'espace compris entre le fond 9f et la face d'extrémité 9e avec la chambre de pompe 8. De plus, la face d'extrémité 9e de la partie cylindrique 2d comporte une siège de clapet 11 qui peut avoir la forme d'un cordon annulaire entourant le débouché du trou 2c sur la face 2e. Enfin, un joint de clapet 10, qui peut être un disque de matériau élastomère, se trouve emprisonné dans l'espace compris entre le fond 9f et la face d'extrémité 2e. Le joint 10 a un diamètre inférieur au diamètre inférieur de la paroi cylindrique 9e. Ainsi, le joint 10 et le siège de clapet 11 forment un clapet d'entrée de la pompe, qui permet à l'air d'entrer à l'intérieur de la chambre de pompe 8 lorsqu'une dépression est créée dans ladite chambre de pompe 8, et qui empêche l'air de sortir de ladite chambre de pompe 8, lorsque ladite chambre de pompe est en surpression puisque le joint 10 est alors appliqué contre le siège de clapet 11 avec étanchéité.

Le ressort de rappel 19 du poussoir 2, auquel il a déjà été fait référence ci-dessus, est monté entre le fond 22a du cylindre de pompe et le rebord annulaire 9b du piston de pompe.

Le corps 1 comporte en outre une paroi cylindrique intérieure 34 concentrique au cylindre de pompe 22 qui s'étend verticalement vers le haut au centre du cylindre de pompe 22, à partir du fond 22a du cylindre 22 jusqu'à une extrémité supérieure 34a. L'extrémité 34a comporte avantageusement une ou plusieurs découpes radiales 34b. La paroi cylindrique 34 définit un cylindre de soupape 13, qui s'étend depuis l'extrémité supérieure 34a de ladite paroi cylindrique 34, jusqu'à une extrémité inférieure 20 débouchant sous le corps 1. L'extémité inférieure 20 du cylindre de soupape 13 peut avantageusement être équipée d'un filtre 21 qui empêche des poussières ou des impuretés de penétrer dans le cylindre de soupape 13. Le filtre 21 peut être un filtre en mousse quelconque, ne créant de préférence qu'une faible perte de charge entre le cylindre de soupape 13 et l'atmosphère. Il peut être fixé au corps 1 par tout moyen : par collage, par serrrage au moyen d'une bague vissée ou soudée au corps 1, etc.

Un piston de soupape 12 coulisse avec étanchéité dans le cylindre de soupape 13, le piston de soupape 12 étant relié au fond 1f du piston de pompe 13 par une tige 12a. Le piston de soupape 13 comporte une face 12b dirigée vers le bas, et une face 12c dirigée vers le haut, qui est en contact avec la chambre de pompe 8.

Lorsqu'un utilisateur actionne un dispositif des figures 1 à 3 en actionnant sur le poussoir 2, le poussoir 2 descend depuis sa position de repos jusqu'à une position dite d'actionnement, qui représente la position de butée basse du poussoir 2. Lorsque le poussoir est dans cette position dite d'actionnement, le piston de soupape 12 se trouve au voisinage du filtre 21, comme représenté en pointillé sur la figure 2. Un orifice de sortie latéral 14 est formé dans le cylindre de soupape 13, juste au-dessus de la position occupée par la face supérieure 12c du piston de soupape 12 lorsque le poussoir est dans sa position d'actionnement. Comme représenté sur la figure 2, l'orifice de sortie 14 fait communiquer le cylindre de soupape 13 avec le canal d'éjection 4.

Ainsi, lorsqu'on actionne le poussoir 2, on comprime le contenu de la chambre de pompe 8, ce qui ferme le clapet d'entrée, puis l'air contenu dans la chambre de pompe 8 est comprimé, jusqu'à ce que la face 12c du piston de soupape ait dégagé l'orifice latéral de sortie 14. L'air contenu dans la chambre de pompe s'échappe alors dans ledit orifice latéral. Dans cette position d'actionnement, les découpes 34b garantissent que le fond 9f du piston n'est pas en contact étanche avec l'extrémié supérieure 34a de le paroi 34, et donc que l'air comprimé contenu dans la chambre de pompe 8 peut s'échapper librement par l'orifice latéral de sortie 14. On notera en outre que l'extrémité inférieure 20 du cylindre de soupape 13 forme un évent, qui évite de créer un flux d'air dans le canal d'éjection avant l'expulsion de l'air comprimé de la chambre de pompe 8, et qui évite aussi de comprimer un volume d'air dans le cylindre de soupape 13 après que la face intérieure 12b du piston de soupape a dépassé l'orifice latéral de sortie 14, ce qui pourrait éventuellement gêner le mouvement de l'ensemble du dispositif.

Lorsqu'on relâche le poussoir 2, le ressort de rappel dudit poussoir 2 ramène le piston de pompe 9 dans sa position intiale. Au cours de ce mouvement, la face supérieure du piston de soupape repasse au-dessus de l'orifice latéral de sortie 14, de sorte que la chambre de pompe 8 est isolée, et qu'une dépression se crée dans la chambre de pompe 8, ce qui ouvre le clapet d'entrée de la pompe. La chambre de pompe se remplit à nouveau d'air jusqu'à ce que le poussoir 2 soit revenu dans sa position de repos.

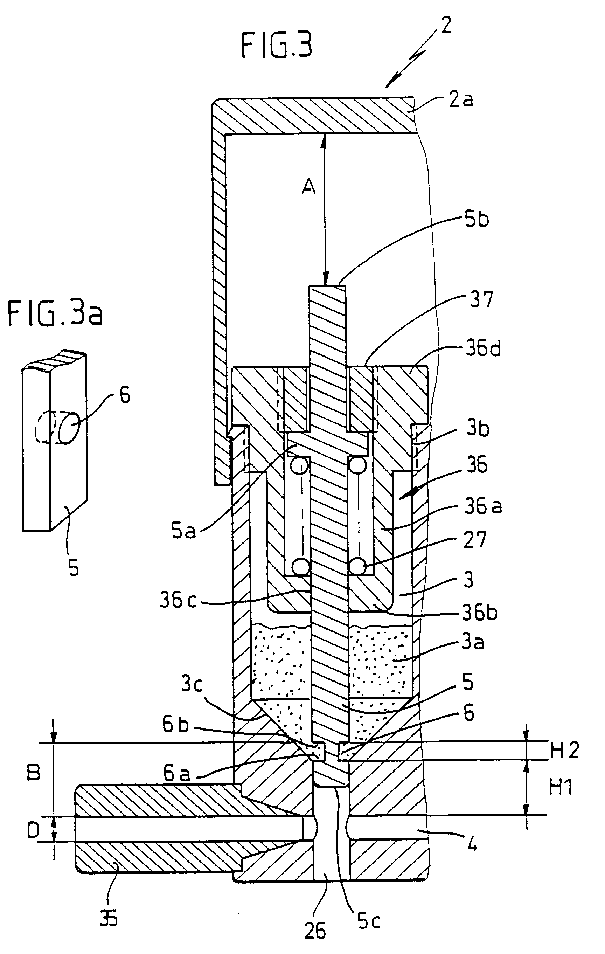

La figure 3 représente les moyens de stockage et de chargement de poudre du dispositif de la figure 1. La réserve de poudre 3 est un logement formé dans le boîtier 1, qui s'étend entre une extrémité supérieure 3b et une extrémité inférieure 3c en forme d'entonnoir. L'extrémité inférieure 3c de la réserve 3 se prolonge verticalement vers le bas par un canal de chargement 26 qui coupe le canal d'éjection 4 à la perpendiculaire, et qui débouche sous le corps 1. Entre l'extrémité inférieure 3c de la réserve 3 et le canal d'éjection 4, le canal de chargement 26 s'étend sur une hauteur H1.

Dans l'extrémité supérieure ouverte 3b de la réserve 3, est montée une coupelle 36 qui comporte une paroi latérale 33 s'étendant verticalement entre une extrémité supérieure ouverte 36d et un fond 36b. Le fond 36b comporte un orifice central 36c. La coupelle 36 est montée dans l'extrémité supérieure 3b de la réserve 3 par tout moyen connu, par exemple par emboîtement en force, par emboîtement et soudage, ou encore par vissage. Dans l'exemple représenté sur la figure 3, l'extrémité supérieure 3b est filetée intérieurement tandis qu'une partie extérieure de la coupelle 36 est filetée extérieurement, la coupelle 36 étant ainsi vissée dans l'extrémité supérieure 3b de la réserve 3.

Une tige de chargement 5, éventuellement de section circulaire, est montée coulissante dans l'orifice 36c de la coupelle et dans le canal vertical 26 de chargement. La tige 5 s'étend verticalemenent entre une extrémité supérieure 5b et une extrémité inférieure 5c. L'extrémité supérieure 5b saille au-dessus de l'extrémité supérieure 36d de la coupelle 36, lorsque le dispositif est dans sa position de repos comme représenté sur la figure 3, tandis que l'extrémité inférieure 5c de la tige de chargement est engagée dans le canal de chargement 26 lorsque le dispositif est dans sa position de repos. La tige 5 comporte en outre un collet 5a, disposé à l'intérieur de la paroi latérale 36a de la coupelle 36. Un ressort 27 de compression est monté entre le collet 5a et le fond 36b de la coupelle 36, sollicitant la tige de chargement 5 vers le haut, c'est-à-dire vers le poussoir 2. Une virole 37 de butée est montée à l'intérieur de l'extrémité supérieure 36b de la coupelle 36, de façon à limiter le mouvement de la tige de chargement 5 vers le haut. La virole 37 peut être fixée par tout moyen connu à la coupelle 36, par exemple emboîtement puis soudage ultrasons, ou encore vissage comme représenté sur la figure 3.

La tige de chargement 5 comporte en outre un évidement de dosage 6, qui dans l'exemple de la figure 3 est un évidement annulaire formé sur la périphérie de la tige 5. L'évidement 6 s'étend verticalement entre un épaulement inférieur 6a et un épaulement supérieur 6b, sur une hauteur H2 inférieure à H1.

Lorsque le poussoir 2 est actionné, il parcourt d'abord une certaine course A avant que sa paroi supérieure 2a ne rencontre la paroi supérieure 5b de la tige de chargement 5. Ensuite, la paroi supérieure 2a du poussoir 2 entraîne la tige de chargement 5 vers la bas, contre la poussée du ressort 27. L'évidement de dosage 6 est donc entrainé vers le canal de chargement 26. L'évidement de dosage 6 est entouré de poudre 3a tant qu'il se trouve dans la réserve 3, et donc il est rempli de poudre 3a. Lorsqu'il pénètre dans la canal de chargement 26, la dose de poudre qu'il contient se trouve isolée à la fois de la réserve 3 et du canal d'éjection 4 du fait de la hauteur H2 de l'évidement est inférieure à la hauteur H1. Puis l'évidement 6 pénètre dans le canal d'éjection 4, de sorte que la dose de poudre de l'évidement 6 n'est plus retenue dans ledit évidement 6. De préférence, la hauteur H2 de l'évidement 6 est inférieure ou égale au diamètre ou à la hauteur D du canal d'éjection 4. De la sorte, l'évidement 6 peut pénétrer en totalité à l'intérieur du canal d'éjection 4. La longueur de la tige 5 est conçue de façon que l'évidement 6 se trouve en totalité dans le canal d'éjection 4 lorsque le poussoir 2 arrive dans sa position d'actionnement et qu'un flux d'air comprimé est crée dans le canal d'éjection 4 par la pompe à air 7. Si B est la distance entre le canal 4 et l'épaulement supérieur 6b qui délimite l'évidement de dosage 6, et si C est la distance verticale entre la face supérieure 12c du piston de soupape 12 et l'orifice latéral de sortie 14 du cylindre de soupape, ledit orifice latéral de sortie ayant généralement un faible diamètre, la distance A + B sera de préférence sensiblement égale à la distance C.

Dans la position d'actionnement, l'extrémité inférieure 5c de la tige de chargement pénètre dans la partie inférieure du canal 26, c'est-à-dire dans la partie du canal 26 située sous le canal d'éjection 4. Comme la tige 5 coulisse avec étanchéité dans le canal 26, le canal d'éjection 4 ne communique donc ni avec la partie inférieure, ni avec la partie supérieure du canal 26 lorsque le poussoir 2 est dans sa position d'actionnement.

Ainsi, lorsque le poussoir 2 arrive dans sa position d'actionnement, et que la pompe 7 crée un flux d'air comprimé dans le canal d'éjection 4 en direction de l'embout 35, le flux d'air comprimé chasse la poudre contenue dans l'évidement de dosage 6 et l'entraine vers l'extérieur, c'est-à-dire dans le cas présent, dans la bouche ou dans le nez d'un patient.

L'évidement de dosage 6 pourrait avoir une forme autre qu'une forme annulaire. Par exemple, comme représenté sur la figure 3a; l'évidement de dosage 6 pourrait être un trou cylindrique, ou éventuellement conique évasé vers l'embout 35, percé dans la tige de chargement 5 parallèlement au canal d'ejection 4. Sur la figure 3a, la tige de chargement 5 a une section rectangulaire, mais ceci n'est pas limitatif, la section de la tige 5 pouvant notamment être circulaire.

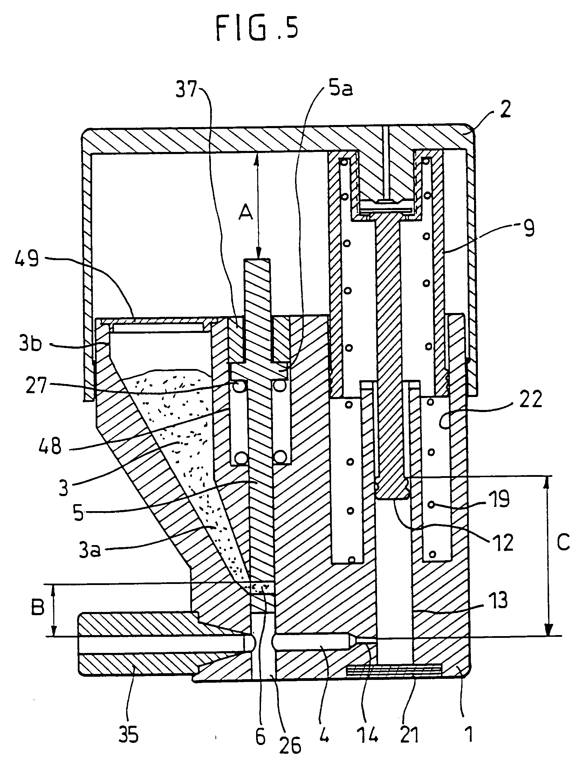

La figure 5 représente une variante du dipositif de la figure 1, qui ne sera pas décrite en détail ici, du fait qu'elle fonctionne de la même façon. A la différence du dispositif de la figure 1, la tige de chargement 5 ne traverse pas la réserve 3 de poudre. Le canal de chargement 26 se prolonge vers le haut jusqu'à un logement 48 qui reçoit le ressort 27 de rappel de la tige 5, et dans lequel est fixée la virole 37 qui sert de butée au collet 5a de la tige. La réserve 3 comporte un couvercle 49 à son extrémité supérieure, et son extrémité inférieure 3a forme un conduit en pente vers le canal 26, et communique avec le canal 26 à la position occupée par l'évidement du stockage 6 lorsque le poussoir 2 est au repos. L'évidement 6 est ici un trou cylindrique parallèle au canal d'éjection 4. Cette variante convient particulièrement à des poudres non susceptibles de s'agglomérer, par exemple des microgranules.

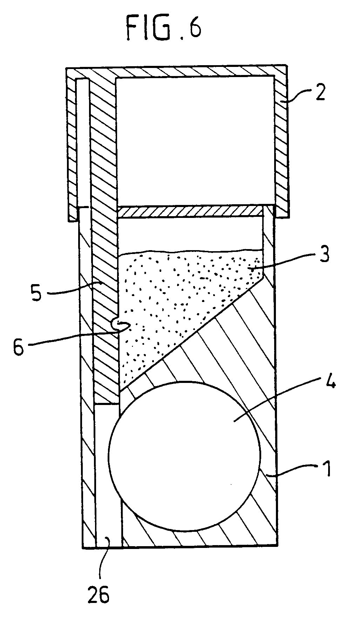

La figure 6 représente une variante du dispositif de la figure 5, dans laquelle la réserve de poudre 3 est disposée non plus sur l'avant du dispositif, mais latéralement. L'évidement de dosage 6 est une saignée droite parallèle au canal d'éjection 4, réalisée seulement sur un coté de la tige de chargement 5. Le canal d'éjection 4 est décalé latéralement par rapport à la tige 5, de sorte que la tige 4 ne pénètre que partiellement dans le canal d'éjection 4. Lorsque le poussoir 2 est dans sa position d'actionnement (abaissée), la saignée 6 de dosage se trouve en totalité dans ledit canal d'éjection 4, de sorte que la poudre qu'elle contient est entraînée par le flux d'air comprimé qui parcourt alors le canal d'éjection 4, et qui est produit par une pompe à air (non représentée) identique à celle des figures 1 et 5.

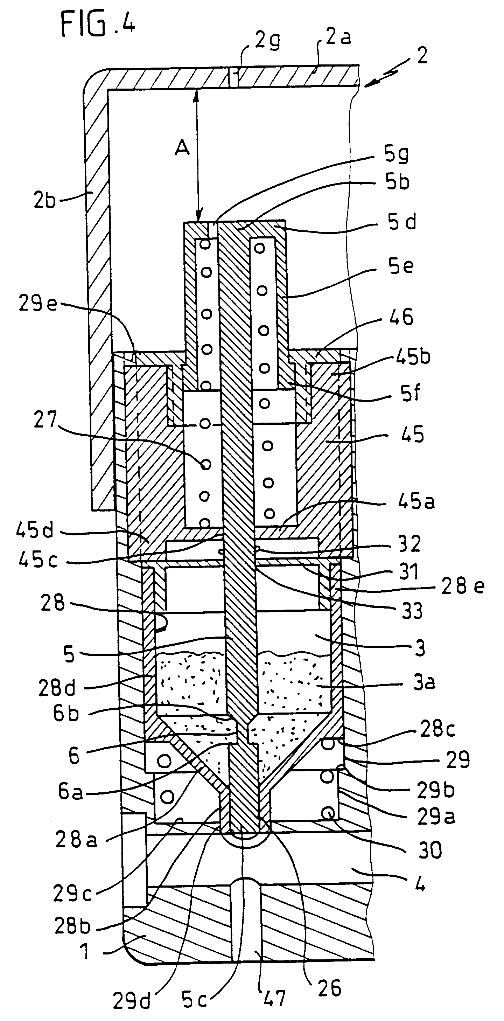

Une variante du système de chargement de dose du dispositif de la figure 1 est représenté plus en détail sur la figure 4. Dans comme l'exemple des figures 1 à 3, le déplacement d'une dose de poudre depuis la réserve 3 jusqu'au canal d'éjection 4 se fait au moyen d'une tige de chargement 5 montée coulissante par rapport au corps 1. Comme précédemment, la tige 5 comporte un évidement de dosage 6, qui est ici annulaire et compris entre un épaulement inférieur 6a et un épaulement supérieur 6b de la tige 5. Dans le cas particulier représenté sur la figure 4, l'épaulement supérieur 6b n'est pas radial, mais forme un angle ouvert, par exemple sensiblement égal à 135° avec la partie rétrécie de la tige qui se trouve au-dessous de lui.

Contrairement à l'exemple des figures 1 à 3, la réserve de poudre 3 n'est pas contituée directement par un logement du corps 1, mais par une enceinte 28, montée coulissante dans un logement cylindrique 29 du corps 1. Le logement 29 s'étend entre une extrémité supérieure ouverte 29e et un fond 29c. Le fond 29c est percé d'un orifice 29d, qui communique avec le canal d'éjection 4. En outre, le logement 29 comporte une partie inférieure 29a de diamètre légèrement réduit qui définit un épaulement annulaire 29b dirigé vers le haut.

L'enceinte 28 comporte une paroi cylindrique 28b, qui coulisse dans le logement 29. La paroi cylindrique 28d s'étend entre une extrémité supérieure 28e et une extrémité inférieure prolongée vers le bas par une paroi conique 28a, elle-même prolongée vers le bas par une paroi cylindrique 28d qui définit un canal de chargement 26. La paroi cylindrique 28b coulisse avec étanchéité dans l'orifice 29d, de sorte qu'elle peut pénétrer au moins partiellement dans le canal d'éjection 4 lorsque l'enceinte 28 coulisse dans son logement 29. En outre, l'enceinte 28 comporte une surface d'appui annulaire 28c extérieure et dirigée vers le bas. Un ressort hélicoïdal 30 de compression est monté entre le fond 29c du logement 29 et la surface d'appui 29c de façon à solliciter l'enceinte 28 vers le haut. En outre, la surface d'appui bute contre l'épaulement 29b lorsque l'enceinte est déplacée suffisamment vers le bas, de sorte que l'épaulement 29 définit une butée basse de l'enceinte 28.

L'extrémité supérieure ouverte 28e de l'enceinte 28 est fermée par un couvercle 31 fixé par tout moyen connu, qui comporte un orifice central 33.

Une coupelle 45, qui comporte une paroi latérale s'étendant axialement entre une extrémité supérieure ouverte 45b et une extrémité inférieure 45d, est montée dans l'extrémité supérieure ouverte 29e du logement 29, par exemple par vissage. La coupelle 45 comporte un fond 45a situé au-dessus de l'extrémité inférieure 45d. Le fond 45a est percé d'un orifice central 45c. En outre, une virole 46 est montée dans l'extrémité supérieure ouverte 45b de la coupelle 45, par exemple par vissage. La tige de chargement 5 s'étend verticalement entre une extrémité inférieure 5c et une extrémité supérieure 5b. Dans la position de repos du poussoir, comme représenté sur la figure 5, l'extrémité inférieure 5c de la tige coulisse avec étanchéité dans le canal de chargement 26 de l'enceinte 28. L'extrémité supérieure 5b de la tige comporte une paroi radiale 5d, qui est ici percée d'un orifice d'évent 5g, et qui se prolonge verticalement vers le bas par une paroi cylindrique 5e, jusqu'à une extrémité supérieure dotée d'un rebord extérieur 5f. Le ressort de rappel 26 de la tige de chargement 5 est disposé entre les fond 45a de la coupelle 45 et la paroi radiale 5d de la tige 5. Le ressort de rappel 27 sollicite la tige 5 vers le haut et la course de la tige 5 vers le haut est limitée par la butée du rebord extérieur 5f contre la virole 46.

Le corps 1 comporte en outre un canal 47 vertical, qui s'étend dans le prolongement de la tige 5, entre le canal d'éjection 4 et le bord inférieur du corps 1.

Enfin, la tige 5 coulisse dans l'orifice 33 du couvercle 31 avec un faible jeu, en tout cas avec un jeu suffisamment faible pour que la poudre contenue dans l'enceinte 28 ne puisse pas s échapper par l'orifice 33. Par ailleurs, la tige 5 comporte un ou plusieurs ergots latéraux 32, qui, lorsque le dispositif est dans sa position de repos, comme représenté sur la figure 6, se trouve entre le fond 45a de la coupelle 45 et le couvercle 31.

Lorsqu'un utilisateur appuie sur la poussoir 2, la paroi supérieure 2a du poussoir 2 parcourt d'abord une certaine course A avant d'entrer en contact avec l'extrémité supérieure 5b de la tige de chargement 5a. Par la suite, l'enfoncement du poussoir 2 entraîne la tige de chargement vers le bas. Les ergots 32 rencontrent donc le couvercle 31, de sorte qu'ils entraînent l'enceinte 28 vers le bas, contre la force du ressort 30. Au cours de ce mouvement, la paroi cylindrique 28b de l'enceinte 28 coulisse avec étanchéité dans l'orifice 29d et pénètre dans le canal d'éjection 4. Dès que la surface d'appui 28c arrive en butée contre l'épaulement 29b, le mouvement de l'enceinte 28 s'arrête. De ce fait, les ergots 32 sont forcés au travers de l'orifice 33 du couvercle 31. Ce passage forcé peut s'effectuer grâce à une certaine souplesse des ergots 32, ou éventuellement grâce à une certaine souplesse du couvercle 31.

A partir de cet instant, le ressort 30 imprime à l'enceinte 28 un brusque mouvement vers le haut, jusqu'à ce que le couvercle 31 vienne buter violemment contre l'extrémité inférieure 45d de la coupelle 45. A ce moment, l'épaulement inférieur 6a de l'évidement de dosage 6 se trouve sensiblement à l'extrémité inférieure de la paroi conique 28a, de sorte que le mouvement brusque et le choc imprimés à l'enceinte 28 entraînent une mise de suspension de la poudre 3a contenue dans l'enceinte 28. Par conséquent, la poudre 3a est désagglomérée, de sorte qu'elle remplit parfaitement l'évidement de dosage 6.

Ensuite, la tige de chargement 5 poursuit son mouvement vers le bas, mais cette fois en coulissant dans le canal de chargement 26 qui joue le même rôle que dans le dispositif des figures 1 à 3. Lorsque l'évidement de dosage 6 se trouve en totalité dans le canal d'éjection 40 le poussoir 2 est en position de poussée basse, c'est-à-dire en position d'actionnement, et l'éjection de l'air comprimé compris dans la chambre de pompe 8 se produit, comme il a été expliqué précédemment. Ainsi, la dose de poudre contenue dans l'évidement de dosage 6 annulaire est explusée par le canal d'éjection 4, sous l'effet du flux d'air comprimé provenant de la chambre de pompe.

Lorsque l'utilisateur relâche le poussoir 2, le poussoir revient en position de repos sous l'effet du ressort de rappel 19, et la tige de chargement 5 revient en position de repos sous l'effet du ressort 27. Au cours de ce mouvement, les ergots 32 sont à nouveau forcés au travers de l'orifice 33 sous l'effet du ressort 27.

Eventuellement, comme représenté en pointillé sur la figure 4, une membrane souple 50 pourrait être fixée de façon étanche sur la tige 5 et le couvercle 31 ou l'enceinte 28, pour améliorer l'étanchéité entre le couvercle 31 et la tige 5.

Dans toutes les formes de réalisation, il serait possible que la tige de chargement 5 soit solidaire du poussoir ou disposée au contact du poussoir, en adaptant la disposition de la tige 5 et/ou le diamètre du piston de pompe.

## Revendications

1. Dispositif pour projeter des doses d'une substance fluide à l'aide d'un flux d'air comprimé, ledit dispositif comprenant :
- un corps (1) ayant un canal d'éjection (4).
- un organe d'actionnement (2) déplaçable par rapport au corps (1) entre une position de repos et une position d'actionnement,
- un organe élastique (19) de rappel de l'organe d'actionnement, qui sollicite l'organe d'actionnement (2) vers sa position de repos,
- une réserve (3) destinée à contenir ladite substance,
- des moyens de chargement (5, 6) adaptés à déplacer une dose de ladite substance hors de la réserve (3) jusque dans le canal d'éjection 4 lorsque l'organe d'actionnement (2) est déplacé de sa position de repos jusqu'à sa position d'actionnement,
- une pompe à air (7) ayant une chambre de pompe (8) dotée d'une paroi déplaçable (9) solidaire de l'organe d'actionnement (2) et adaptée à comprimer la chambre de pompe (8) lorsque l'organe d'actionnement est déplacé depuis sa position de repos vers sa position d'actionnement, ladite chambre de pompe (8) ayant un clapet de sortie (12, 13, 14) qui communique avec le canal d'éjection (4), le clapet de soitie étant constitué par un organe d'obturation (12) collaborant avec une ouverture (14) de sortie, ledit organe d'obturation (12) isolant l'ouverture de sortie (14) par rapport à la chambre de pompe (8) tant que l'organe d'actionnement (2) n'est pas au voisinage immédiat de sa position d'actionnement, ledit organe d'obturation (12) ouvrant la communication entre la chambre de pompe (8) et l'ouverture de sortie (14) lorsque l'organe d'actionnement (2) est au voisinage immédiat de sa position d'actionnement,
caractérisé en ce que ladite chambre de pompe (8) a un clapet d'entrée (10, 11) qui communique avec l'atmosphère et en ce que l'organe d'obturation est constitué par un piston de soupape (12) solidaire en déplacement de l'organe d'actionnement (2) et coulissant avec étanchéité dans un cylindre de soupape (13) ayant une extrémité supérieure (34a), ledit cylindre ayant une ouverture de sortie (14) qui communique avec le canal d'éjection (4), ledit cylindre de soupape ayant son extrémité (34a) qui communique avec la chambre de pompe (8), ladite ouverture de sortie étant positionnée suffisamment éloignée de ladite extrémité (34a) du cylindre de soupape pour que le piston de soupape (12) isole l'ouverture (14) de sortie par rapport à la chambre de pompe tant que l'organe d'actionnement (2) n'est pas au voisinage immédiat de sa position d'actionnement.

2. Dispositif selon la revendication 1, dans lequel le cylindre de soupape (13) possède un orifice d'évent (20) positionné de façon que ladite ouverture de sortie (14) soit disposée entre ledit orifice d'évent et ladite extrémité (34a) du cylindre de soupape qui communique avec la chambre de pompe (8).

3. Dispositif selon la revendication 2, dans lequel ledit orifice d'évent (20) est muni d'un filtre (21) pour empêcher la pénétration de poussières dans le cylindre de soupape.

4. Dispositif selon l'une quelconque des revendications 1 à 3, dans lequel les moyens de chargement (5, 6) sont constitués par une tige (5) de chargement, ayant un évidement de dosage (6) à une position intermédiaire sur sa longueur, la tige de chargement n'est pas solidaire de l'organe d'actionnement (2), ladite tige de chargement est sollicitée vers une position de butée par un moyen élastique (27) et l'organe d'actionnement parcourt une certaine course (A) depuis sa position de repos avant de repousser ladite tige contre l'action du moyen élastique (27) de la tige de chargement.

5. Dispositif selon la revendication 4, dans lequel l'évidement de dosage a une forme annulaire et se trouve compris dans le canal d'éjection lorsque l'organe d'actionnement est dans sa position d'actionnement.

6. Dispositif selon l'une quelconque des revendications 1 à 4, dans lequel la tige de chargement (5) coulisse dans la réserve (3) de substance à projeter, ladite réserve (3) est une enceinte (28) coulissant à mouvement perdu parallèlement à la tige de chargement (5) dans un logement (29) du corps (1), un moyen élastique (30) sollicite ladite enceinte (28) de façon à l'éloigner du canal d'éjection (4), l'enceinte possède une paroi (31) éloignée du canal d'éjection (4), qui comporte un orifice (33) traversé par la tige de chargement (5), la tige de chargement (5) comporte un relief latéral (32) qui se trouve à l'extérieur de l'enceinte (28) lorsque l'organe d'actionnement (2) est dans sa position de repos, ledit relief latéral (32) interfère avec ladite paroi (31) de l'enceinte en déplaçant l'enceinte sur une certaine distance contre la sollicitation du moyen élastique (30) de l'enceinte lorsque l'organe d'actionnement (2) est déplacé vers sa position d'actionnement, et ledit relief latéral (32) a une souplesse suffisante pour s'effacer élastiquement en traversant ledit orifice (33) de ladite paroi (31) de l'enceinte, lorsqu'une force axiale suffisante est exercée par ledit relief latéral (32) sur ladite paroi (31) de l'enceinte.

7. Dispositif selon l'une quelconque des revendications 1 à 4, dans lequel la tige de chargement (5) coulisse dans la réserve (3) de substance à projeter, ladite réserve (3) est une enceinte (28) coulissant à mouvement perdu parallèlement à la tige de chargement (5) dans un logement (29) du corps (1), un moyen élastique (30) sollicite ladite enceinte (28) de façon à l'éloigner du canal d'éjection (4), l'enceinte possède une paroi (31) éloignée du canal d'éjection (4), qui comporte un orifice (33) traversé par la tige de chargement (5), la tige de chargement (5) comporte un relief latéral (32) qui se trouve à l'extérieur de l'enceinte (28) lorsque l'organe d'actionnement (2) est dans sa position de repos, ledit relief latéral (32) interfère avec ladite paroi (31) de l'enceinte en déplaçant l'enceinte sur une certaine distance contre la sollicitation du moyen élastique (30) de l'enceinte lorsque l'organe d'actionnement (2) est déplacé vers sa position d'actionnement, et ladite paroi (31) de l'enceinte (28), qui est éloignée du canal d'éjection, a une souplesse suffisante pour se déformer élastiquement de façon à laisser le relief latéral (32) de la tige de chargement traverser l'orifice (33) de ladite paroi (31) lorsqu'une force axiale suffisante est exercée par ledit relief latéral (32) sur ladite paroi (31) de l'enceinte.

## Claims

1. A device for projecting measured quantities of a fluid substance by means of a puff of compressed air, said device comprising:
a body (1) having an ejection channel (4);
an actuator member (2) displaceable relative to the body (1) between a rest position and an actuated position;
a resilient return member (19) for the actuator member, urging the actuator member (2) towards its rest position;
a supply (3) for containing said substance;
feed means (5, 6) adapted to displace a measured quantity of said substance out of the supply (3) into the ejection channel (4) when the actuator member (2) is displaced from its rest position to its actuated position; and
an air pump (7) having a pump chamber (8) provided with a displaceable wall (9) secured to the actuator member (2) and adapted to compress the pump chamber (2) when the actuator member is displaced from its rest position to its actuated position, said pump chamber (8) having an inlet valve (10, 11) which communicates with the atmosphere, and an outlet valve (12, 13, 14) which communicates with the ejection channel (4), the outlet valve being constituted by a shutter member (12) which co-operates with an outlet opening (14), said shutter member (12) isolating the outlet opening (14) from the pump chamber (8) so long as the actuator member (2) is not in the immediate vicinity of its actuated position, said shutter member (12) opening communication between the pump chamber (8) and the outlet opening (14) when the actuator member (2) is in the immediate vicinity of its actuated position;
the device being characterized in that said pump chamber (8) has an inlet valve (10, 11) which communicates with the atmosphere and that the outlet valve is constituted by a valve piston (12) constrained to move with the actuator member (2) and sliding in sealed manner in a valve cylinder (13) having a top end (34a), said cylinder having a lateral outlet orifice (14) which communicates with the ejection channel (4), said valve cylinder having its top end (34a) in communication with the pump chamber (8), said lateral outlet orifice being positioned sufficiently far from said top end (34a) of the valve cylinder for the valve piston (12) to isolate the lateral outlet orifice (14) from the pump chamber so long as the actuator member (2) is not in the immediate vicinity of its actuated position.

2. A device according to claim 1, in which the valve cylinder (13) possesses a vent orifice (20) positioned in such a manner that said lateral outlet orifice (14) is disposed between said vent orifice and said top end (34a) of the valve cylinder which is in communication with the pump chamber (8).

3. A device according to claim 2, in which said vent orifice (20) is provided with a filter (21) to prevent dust penetrating into the valve cylinder.

4. A device according to any one of claims 1 to 3, in which the feed means (5, 6) are constituted by a feed rod (5) having a measuring recess (6) at an intermediate position along its length, the feed rod not being secured to the actuator member (2), said feed rod being urged towards an abutment position by resilient means (27) and the actuator member travelling along a certain stroke (A) from its rest position before pushing said rod against the action of the resilient means (27) of the feed rod.

5. A device according to claim 4, in which the measuring recess is annular in shape and is located within the ejection channel when the actuator member is in its actuated position.

6. A device according to any one of claims 1 to 4, in which the feed rod (5) slides in the supply (3) of substance to be projected, said supply (3) is an enclosure (28) that slides with lost motion parallel to the feed rod (5) in a housing (29) of the body (1), resilient means (30) urging said enclosure (28) away from the ejection channel (4), the enclosure possessing a wall (31) remote from the ejection channel (4), which wall includes an orifice (33) through which the feed rod (5) passes, the feed rod (5) including a lateral projection (32) that is outside the enclosure (28) when the actuator member (2) is in its rest position, said lateral projection (32) interfering with said wall (31) of the enclosure by displacing the enclosure a certain distance against the urging of the resilient means (30) of the enclosure when the actuator member (2) is displaced towards its actuated position, and said lateral projection (32) is sufficiently flexible to retract resiliently as it passes through said orifice (33) of said wall (31) of the enclosure once sufficient axial force is exerted by said lateral projection (32) on said wall (31) of the enclosure.

7. A device according to any one of claims 1 to 4, in which the feed rod (5) slides in the supply (3) of substance to be projected, said supply (3) is an enclosure (28) that slides with lost motion parallel to the feed rod (5) in a housing (29) of the body (1), resilient means (30) urging said enclosure (28) away from the ejection channel (4), the enclosure possessing a wall (31) remote from the ejection channel (4), which wall includes an orifice (33) through which the feed rod (5) passes, the feed rod (5) including a lateral projection (32) that is outside the enclosure (28) when the actuator member (2) is in its rest position, said lateral projection (32) interfering with said wall (31) of the enclosure by displacing the enclosure a certain distance against the urging of the resilient means (30) of the enclosure when the actuator member (2) is displaced towards its actuated position, and said wall (31) of the enclosure (28) which is remote from the ejection channel is sufficiently flexible to deform elastically so as to allow the lateral projection (32) of the feed rod to pass through the orifice (33) of said wall (31) when sufficient axial force is exerted by said lateral projection (32) on said wall (31) of the enclosure.

## Patentansprüche

1. Vorrichtung zum Herausschleudern von Dosissen eines Fluids mit Hilfe eines Stromes von komprimierter Luft, wobei diese Vorrichtung folgende Bestandteile umfaßt:
- einen Körper (1), der einen Ausstoßkanal (4) besitzt,
- ein Betätigungsorgan (2), das bezüglich des Körpers (1) zwischen einer Ruhelage und einer Arbeitsstellung verschiebbar ist,
- ein elastisches Rückholorgan (19) für das Betätigungsorgan, das das Betätigungsorgan (2) in seine Ruhelage vorspannt,
- einen Behälter (3), der dazu dient, die Substanz aufzunehmen,
- Ladeeinrichtungen (5, 6), die geeignet sind, eine Dosis der Substanz aus dem Behälter (3) bis in den Ausstoßkanal (4) zu verlagern, wenn das Betätigungsorgan (2) aus seiner Ruhelage in seine Arbeitsstellung verschoben wird,
- eine Luftpumpe (7), die eine Pumpenkammer (8) aufweist, welche mit einer verschiebbaren Wand (9) versehen ist, die mit dem Betätigungsorgan (2) fest verbunden und geeignet ist, die Pumpenkammer (8) zusammenzudrücken, wenn das Betätigungsorgan aus seiner Ruhelage heraus zu seiner Betätigungsstellung hin verschoben wird, wobei die Pumpenkammer (8) ein Austrittsventil (12, 13,14) aufweist, das mit dem Ausstoßkanal (4) in Verbindung steht, wobei das Ausgangsventil von einem Verschlußorgan (12) gebildet wird, das mit einer Ausgangsöffnung (14) zusammenwirkt, wobei das Verschlußorgan (12) die Ausgangsöffnung (14) bezüglich der Pumpenkammer (8) verschließt, solange das Betätigungsorgan (2) sich nicht in unmittelbarer Nachbarschaft seiner Arbeitsstellung befindet, und wobei das Verschlußorgan (12) die Verbindung zwischen der Pumpenkammer (8) und der Ausgangsöffnung (14) öffnet, wenn sich das Betätigungsorgan (2) in unmittelbarer Nachbarschaft seiner Arbeitsstellung befindet,
dadurch gekennzeichnet, daß die Pumpenkammer (8) ein Eintrittsventil (10, 11) aufweist, das mit der Atmosphäre in Verbindung steht, und daß das Verschlußorgan von einem Ventilkolben (12) gebildet wird, der verschiebungsfest mit dem Betätigungsorgan (2) verbunden ist und dicht in einem Ventilzylinder (13) gleitet, der ein oberes Ende (34a) aufweist, wobei der Zylinder eine Ausgangsöffnung (14) besitzt, die mit dem Ausstoßkanal (4) in Verbindung steht, und wobei der Ventilzylinder an seinem Ende (34a) mit der Pumpenkammer (8) in Verbindung steht, wobei die Ausgangsöffnung genügend weit von dem besagten Ende (34a) des Ventilzylinders weg angeordnet ist, so daß der Ventilkolben (12) die Ausgangsöffnung (14) bezüglich der Pumpenkammer absperrt, solange sich das Betätigungsorgan (2) nicht in unmittelbarer Nähe seiner Arbeitsposition befindet.

2. Vorrichtung nach Anspruch 1, bei der der Ventilzylinder (13) eine Belüftungsöffnung (20) aufweist, die derart positioniert ist, daß sich die Ausgangsöffnung (14) zwischen der Belüftungsöffnung und dem Ende (34a) des Ventilzylinders befindet, das mit der Pumpenkammer (8) in Verbindung steht.

3. Vorrichtung nach Anspruch 2, bei der die Belüftungsöffnung (20) mit einem Filter (21) versehen ist, um das Eindringen von Staub in den Pumpenzylinder zu verhindern.

4. Vorrichtung nach einem der Ansprüche 1 bis 3, bei der die Ladeeinrichtungen (5, 6) von einer Ladestange (5) gebildet werden, die eine Dosiervertiefung (6) an einer mittleren Stelle ihrer Länge aufweist, wobei die Ladestange nicht fest mit dem Betätigungsorgan (2) verbunden ist, wobei die Ladestange durch ein elastisches Element (27) gegen eine Anschlagsposition vorgespannt ist und das Betätigungsorgan einen bestimmten Weg (A) aus seiner Ruhelage durchläuft, bevor es die Stange gegen die Wirkung des elastischen Elements (27) der Ladestange zurückschiebt.

5. Vorrichtung nach Anspruch 4, bei der die Dosierausnehmung eine Ringform besitzt und im Ausstoßkanal aufgenommen ist, wenn sich das Betätigungsorgan in seiner Arbeitsstellung befindet.

6. Vorrichtung nach einem der Ansprüche 1 bis 4, bei der die Ladestange (5) in den Behälter (3) der abzugebenden Substanz gleitet, wobei der Behälter (3) ein umschlossener Raum (28) ist, der über einen toten Weg parallel zur Ladestange (5) in einer Ausnehmung (29) des Körpers (1) gleitet, wobei ein elastisches Element (30) den umschlossenen Raum (28) in der Weise vorspannt, daß sie ihn vom Ausstoßkanal (4) entfernt hält, wobei der umschlossene Raum eine vom Ausstoßkanal (4) entfernt liegende Wand (31) aufweist, die eine Öffnung (33) umfaßt, durch die hindurch sich die Ladestange (5) erstreckt, wobei die Ladestange (5) wenigstens einer seitlichen Erhebung (32) aufweist, die sich außerhalb des umschlossenen Raums (28) befindet, wenn sich das Betätigungsorgan (2) in seiner Ruhelage befindet, wobei die seitliche Erhebung (32) mit der Wand (31) des umschlossenen Raums in der Weise wechselwirkt, daß sie den umschlossenen Raum über einen gewissen Abstand gegen die Vorspannung des elastischen Elements (30) des umschlossenen Raums verschiebt, wenn das Betätigungsorgan (2) zu seiner Arbeitsstellung hin verschoben wird, und wobei die wenigstens eine seitliche Erhebung (32) eine ausreichende Nachgiebigkeit besitzt, um in elastischer Weise auszuweichen, um durch die Öffnung (33) der Wand (31) des umschlossenen Raums hindurchzutreten, wenn eine ausreichend große axiale Kraft durch die seitliche Erhebung (32) auf die Wand (31) des umschlossenen Raums ausgeübt wird.

7. Vorrichtung nach einem der Ansprüche 1 bis 4, bei der die Ladestange (5) im Behälter (3) für die auszustoßende Substanz gleitet, der Behälter (3) ein umschlossener Raum (28) ist, der über einen toten Hubweg parallel zur Ladestange (5) in einer Ausnehmung (29) des Körpers (1) gleitet, wobei ein elastisches Element (30) den umschlossenen Raum (28) vom Ausstoßkanal (4) weg vorspannt, wobei der umschlossene Raum eine Wand (31) umfaßt, die vom Ausstoßkanal (4) entfernt ist, und eine Öffnung (33) umfaßt, durch die sich die Ladestange (5) hindurch erstreckt, wobei die Ladestange (5) zeitliche Erhebungen (32) aufweist, die sich außerhalb des umschlossenen Raums (28) befinden, wenn sich das Betätigungsorgan (2) in seiner Ruhelage befindet, wobei die seitlichen Erhebungen (32) mit der Wand (31) des umschlossenen Raums in der Weise wechselwirken, daß sie den umschlossenen Raum über einen gewissen Weg gegen die Vorspannung des elastischen Elements (30) für den umschlossenen Raum verschieben, wenn das Betätigungsorgan (2) zu seiner Arbeitsposition hin verschoben wird, wobei die Wand (31) des umschlossenen Raums (28), die vom Ausstoßkanal entfernt angeordnet ist, eine ausreichende Nachgiebigkeit besitzt, um sich in elastischer Weise derart zu verformen, daß sie die seitlichen Erhebungen (32) der Ladestange durch die Öffnung (33) der Wand (31) hindurchtreten läßt, wenn eine ausreichende axiale Kraft von den seitlichen Erhebungen (32) auf die Wand (31) des umschlossenen Raums ausgeübt wird.
